# EUROPEAN PATENT APPLICATION

(11) **EP 4 461 276 A1**
(43) Date of publication of application: **13.11.2024**
(21) Application number: 24174766.6
(22) Date of filing: 08.05.2024
(51) Int. Cl.: A61F 7/00, A61B 17/00

(54) **DYNAMIC USER COOLING INPUT FOR AN ELECTRIC WHOLE-BODY CRYOTHERAPY CHAMBER**

(30) Priority: 08.05.2023 US 202363464713 P
(71) Applicant: Cryofile LLC dba Artica Systems, Beaverton, Oregon 97003 (US)
(72) Inventor: Kuklinski, Evan, Beaverton, 97003 (US)
(74) Representative: Stevens Hewlett & Perkins

(57) **Abstract**

A cryotherapy chamber with one or more integrated systems. A first system is a prescriptive windchill system that incorporates at least one auxiliary windchill fan located within the cryotherapy chamber that is in signal communication with a user interface also located and accessible within the cryotherapy chamber that responds to control signals provided by a user inside of the cryotherapy chamber to cause the at least one auxiliary windchill fan to activate and to control a speed to further lower a temperature of the cryotherapy chamber. A second system includes a presence sensor that detects a presence of a user when located within the cryotherapy chamber and indicates when the user is no longer present in the cryotherapy chamber to allow an administrative entity to externally turn down or turn off one or more systems in the cryotherapy chamber when the user exits the cryotherapy chamber.

## Description

### CROSS-REFERENCE TO RELATED APPLICATIONS

This application is a non-provisional application, which claims priority to U.S. Provisional Patent Application No. 63/464,713 filed on May 8, 2023, which is incorporated by reference in its entirety.

### FIELD OF THE DISCLOSURE

The present invention pertains to enhancements in the technology and operational systems of cryotherapy chambers. Key advancements include the integration of a prescriptive windchill feature that allows users to customize their experience by adjusting the perceived air temperature within the chamber. This customization is achieved through dynamic control over several variables: the temperature setting, the speed of evaporator fans, and the flow rate of the compressor, which together modulate the chamber's cooling intensity. Additionally, auxiliary windchill fans are employed to further tailor the cooling effect based on the user's preference.

The system also incorporates a presence detection sensor designed to recognize when a user occupies the chamber, ensuring that the system operates only when needed, thereby enhancing safety and energy efficiency. Another notable feature is the cryotherapy boost system, which includes a variable frequency drive (VFD). This component boosts the refrigeration system's cooling capacity and significantly reduces the time required to prepare the chamber for the next user, minimizing wait times and enhancing user throughput. These innovations collectively aim to provide a more controlled, efficient, and user-responsive cryotherapy experience.

### BACKGROUND

Cryotherapy or cold therapy is often used for healing purposes to help those with muscle recovery and overall wellness therapy. Whole body cryotherapy (WBC) involves sitting or standing in a small, enclosed chamber (also known as cryotherapy chamber or a cryochamber) that has been cooled to an extremely low temperature. The entire body is exposed to frigid temperatures which may range from -90 degrees Fahrenheit or lower (including up to - 160 or -200 degrees Fahrenheit other numbers to suit the user).

Cryotherapy involves exposing the body to extremely cold temperatures for a short period, typically in a cryotherapy chamber. While the surface skin temperature can decrease significantly during this exposure, the core body temperature is generally only minimally affected. This rapid cooling of the skin triggers a physiological response where the blood vessels constrict initially and then dilate, a process known as reactive vasodilation. This response helps to increase blood circulation and may temporarily elevate the oxygen and nutrient supply to the peripheral tissues, which some users report helps in reducing pain and inflammation, particularly after physical activity.

Currently, problems still exist with cryotherapy chambers with respect to enabling a user to adjust how cold it is within the cryotherapy chamber while the user is inside the cryotherapy chamber. Notably, every cryotherapy chamber currently has a predefined temperature driven or set by manufacturers and not the user. Typically, there is no such controller that offers the user the ability to control the temperature and to make adjustments to raise or lower the temperature within the cryotherapy chamber. Additionally, when multiple users need to be able to access a cryotherapy chamber in a short amount of time, (e.g., back to back with minimal wait time), the rebound time for a cryotherapy chamber to cool all the way down to the desired temperature is longer than desirable. For example, for athletic teams or sports teams that require a cryotherapy chamber to treat their players before a game or practice within a short period of time, each user emits body heat while in the cryotherapy chamber that causes the cryotherapy chamber to increase its temperature and it takes some duration of time to ensure that the cryotherapy chamber returns to its coldest temperature or setting desired by the next user.

Accordingly, there is still a need for an improved system and process for cryotherapy chambers to address the above existing deficiencies.

### SUMMARY

The present description includes one or more non-limiting embodiments for a system, comprising a cryotherapy chamber. The cryotherapy chamber comprises a cryoevaporator comprising at least one evaporator fan. The prescriptive windchill system comprising of at least one auxiliary windchill fan coupled to a user interface controller, wherein the user interface controller is accessible to a user from within an interior of the cryotherapy chamber and wherein the at least one auxiliary windchill fan is located within the interior of the cryotherapy chamber, wherein the user interface controller is selectable by the user to automatically activate and adjust a level of speed of the at least one auxiliary windchill fan or at least one evaporator fan. The external engine comprising a programmable logic controller (PLC) that controls and is in signal communication and in electronic communication with the user interface controller. The system includes a presence sensor system comprising at least one presence sensor positioned within the cryotherapy chamber, wherein if the at least one presence sensor detects a vacancy inside of the cryotherapy chamber, a signal is sent to the PLC to return the at least one auxiliary windchill fan to a default state that comprises reducing a speed of the at least one auxiliary windchill fan or turns of the at least one auxiliary windchill fan. The system also comprises a cryotherapy boost system, the cryotherapy boost system further comprising a variable frequency drive (VFD) that causes the flowrate of refrigerant to increase through a heat transfer system that is part of an operation of the cryotherapy chamber, thereby increasing an amount of heat that is extracted from the cryochamber per second. The heat transfer system comprises a heat exchanger, one or more exchange valves, a low stage compressor, a high stage compressor, and at least one condenser. The mass flow rate of the refrigerant during operation of the cryotherapy boost system is greater than a mass flow rate of the refrigerant of the low stage compressor. The presence sensor is an infrared sensor that detects infrared heat of the user. The user interface is a frictional slide potentiometer. The activation of the at least one auxiliary windchill at a minimum level further activates the cryotherapy boost system to achieve a lower temperature within the cryotherapy chamber. The system further includes at least one presence sensor is positioned inside of the cryotherapy chamber near a door near the entrance and/or exit of the cryotherapy chamber. The at least one presence sensor may alternatively be positioned on the floor sensor positioned on a floor of the cryotherapy chamber . The user interface controller may comprise knobs, buttons, and/or sliders, and/or smart watches, tablets, and/or digital interfaces.

The present description further includes non-limiting embodiments describing a cryotherapy chamber comprising a cryoevaporator comprising at least one evaporator fan and a prescriptive windchill system. The prescriptive windchill system comprises at least one auxiliary windchill fan coupled to a user interface controller, wherein the user interface controller is accessible to a user from within an interior of the cryotherapy chamber and wherein the at least one auxiliary windchill fan is located within the interior of the cryotherapy chamber, wherein the user interface controller is selectable by the user to automatically activate and adjust a level of speed of the at least one auxiliary windchill fan and the at least one evaporator fan.

Other aspects and advantages of the invention will be apparent from the following description and the appended claims.

### BRIEF DESCRIPTION OF THE DRAWINGS

Embodiments of the present disclosure are described in detail below with reference to the following drawings. These and other features, aspects, and advantages of the present disclosure will become better understood with regard to the following description, appended claims, and accompanying drawings. The drawings described herein are for illustrative purposes only of selected embodiments and not all possible implementations and are not intended to limit the scope of the present disclosure.
FIG. 1 is a block diagram of a system for a cryotherapy chamber having unique functions associated with prescriptive windchill, presence sensor, cryotherapy boost systems, rapid defrost, and artificial intelligence and diagnostics systems.
FIG. 2 is a diagram for a system that includes a presence sensor system for detecting users when located within the cryotherapy chamber.
FIG. 3 is a diagram for a system that includes a prescriptive windchill system incorporated within a cryotherapy chamber.
FIG. 4 is a pictorial illustration of cryotherapy boost system operational with a cryotherapy chamber.
FIG. 5 is a pictorial illustration of an exterior of a cryotherapy chamber.
FIG. 6 is a pictorial illustration of a cryoevaporator that is part of the cryotherapy chamber.
FIG. 7 is a pictorial illustration of evaporator fans and auxiliary windchill fans located proximate to the evaporator fans.
FIG. 8 is a pictorial illustration of a user interface inside of the cryotherapy chamber for controlling and operating the auxiliary windchill fans.
FIG. 9 is a pictorial illustration of an exterior view of an engine having one or more controllers, compressors, as well as a variable frequency drive (VFD) controlling a cryotherapy boost system of the cryotherapy chamber.
FIG. 10 is a pictorial illustration of an internal view of the engine shown in FIG. 10.
FIG. 11 is a pictorial illustration of the engine in fluid communication via one or more pipes with the condensing unit positioned outside of the cryotherapy chamber.
FIG. 12 is a pictorial illustration of the high exchange condenser located outside of the cryotherapy chamber.

### DETAILED DESCRIPTION

The present description includes embodiments for an improved cryotherapy chamber. Additional details are provided with respect to the Figures.

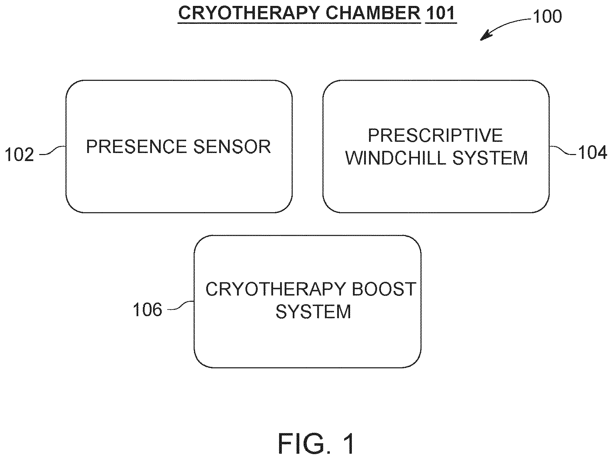

FIG. 1 provides a block diagram for a cryotherapy system 100 comprising a cryotherapy chamber 101 having a number of unique and novel functions and features, including but not limited to, a presence sensor system 102, prescriptive windchill system 104, and cryotherapy boost system 106.

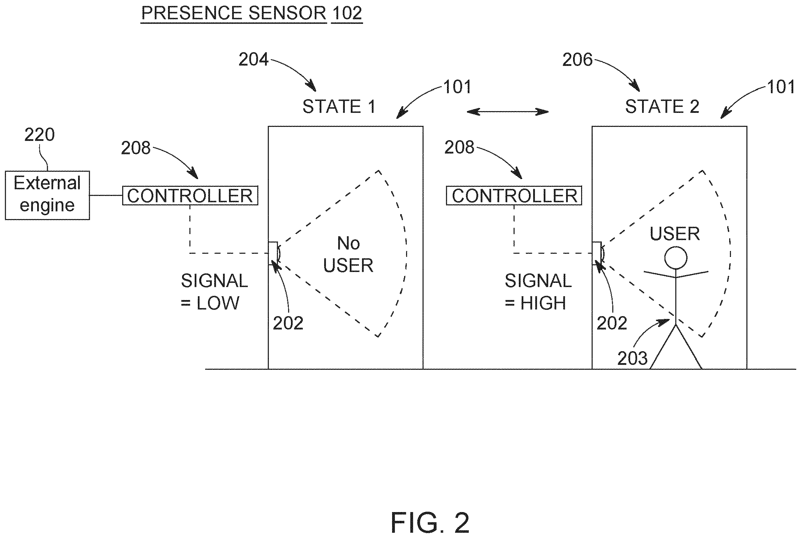

In a non-limiting embodiment, the presence sensor system 102 addresses a problem that currently exists with cryotherapy chambers which is that there is no way of determining when a user is located inside of the cryotherapy chamber 101 for use in logic that is necessary for temperature control and/or safety measures that the administrative staff or control system would like to be able to implement in order to stop or end cryotherapy treatment and impose therapy duration limits. FIG. 2 illustrates and discloses one exemplary embodiment of a presence sensor system 102 that may be applied to a cryotherapy chamber 101.

It is noted that the term "controller" as used herein for any of the systems including the system for the presence sensor 102, prescriptive windchill system 104, and cryotherapy boost system 106 may comprise any type of user interface controller. Such user interface controllers may provide a variety of mechanisms and means to enter an input and trigger an output from the user interface controller. Such user interface controllers may include, but are not limited, to one or more consoles or plates or bases having one or more manually adjustable mechanisms that include but are not limited to knobs, buttons, sliders. Further, the user interface controller may include one or more smartwatches, tablets, computers, smartphones, smart goggles or glasses, remote controllers, or other means of controlling a particular function or application, including but not limited to providing an input to effect a change to the cryotherapy chamber 101 via the presence senses

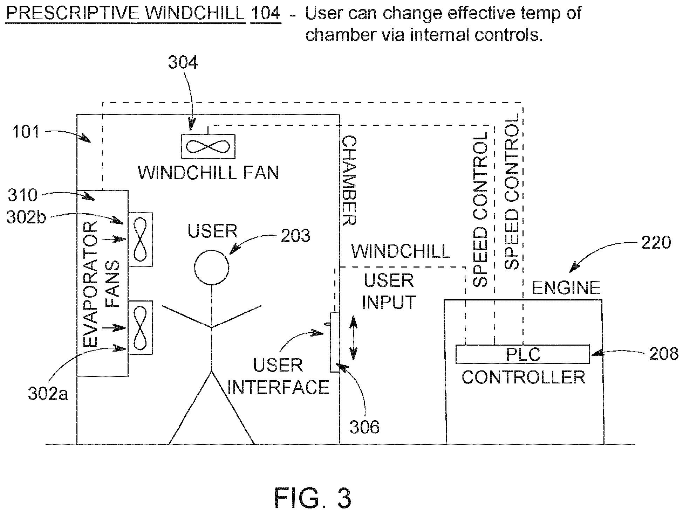

The prescriptive windchill system 104 addresses a problem that currently exists with the fact that the user is not able to control the intensity of the cold therapy treatment and/or how cold the temperature of the cryotherapy chamber feels while the user is located inside of the cryotherapy chamber 101. Currently, cryotherapy chambers typically include one or more evaporator fans (e.g., evaporator fans 302a and 302b as shown in FIG. 3) located within the cryotherapy chamber 101 that operate at a constant flow rate or allow regulation via controls that lie outside of the cryotherapy chamber 101. Having controls located externally to the cryotherapy chamber 101 may be due in part because the temperatures are so cold within the cryotherapy chamber 101 that many controllers and systems and interfaces fail and are not operational if not carefully engineered to withstand the extremely cold temperatures. Notwithstanding the above, the prescriptive windchill system 104 is designed to have auxiliary windchill fans 304 (e.g., as shown in FIG. 3) located within the cryotherapy chamber 101 that are controllable by a user interface/controller 306 accessible to the user while inside of the cryotherapy chamber 101 such that the user is able to be prescriptive of the user's therapy by dynamically changing the effective air temperature of the cryotherapy chamber 101 during the user's session. Further details are provided with respect to FIG.3 and FIGS.5-8.

With respect to cryotherapy boost system 106, cryotherapy boost system 106 may facilitate in reducing rebound time, which is the amount of time needed for a cryotherapy chamber 101 to recover to a set ultra-low temperature after a user has utilized the cryotherapy chamber 101 for a treatment session and concluded the treatment session, and another user desires to use the cryotherapy chamber 101. Every user emits body heat which can raise the temperature of the cryotherapy chamber 101 to some degree. When a first user enters a cryotherapy chamber 101, static heat leak and dynamic heat load is increased. If multiple users want to use a cryotherapy chamber 101 back to back with very little wait time between each user (which may also be referred to herein as "concurrent use" or "successive use" or "use in succession"), such as for instance for a team of athletes after a game or practice that need the benefits of a cold therapy treatment in a cryotherapy chamber 101, it is very difficult to do so in a timely and efficient manner because each user has to wait during the rebound time, or the time required to have the cryotherapy system 100 allow the cryotherapy chamber 101 to achieve the desired temperature for the next user and extract the amount of heat added by a previous user to the cryotherapy chamber 101. Accordingly, the cryotherapy boost system 106 may assist in dynamically changing the steady state capacity of the cryotherapy system 100 to mitigate temperature deltas or changes through the use of variable frequency drives (VFDs) and high stage vapor injection (in some instances) to allow the cryotherapy system 100 to ramp up performance during use. In a non-limiting embodiment, the system is further aided by the combination of the cryotherapy boost system 106 with the presence sensor system 102 and the prescriptive windchill system 104.

In a non-limiting embodiment, the cryotherapy chamber 101 may further benefit from the inclusion of a rapid defrost system 100. It is known that water vapor ingress into a cryotherapy system 100 is inevitable due to the inherent moisture in the air, which may contribute to about 20% of the overall water vapor ingress into the cryotherapy chamber 101. Perspiration and respiration from the user during a session in the cryotherapy chamber 101 contributes about 80% of the moisture in the air in the cryotherapy chamber 101. This moisture in the air nucleates as ice on the evaporator coil leading to a steep decrease in efficiency and performance. Clearing the ice from the evaporator traditionally requires warming of the entire cryotherapy chamber 101 using electric heaters and long durations (e.g., 2-3 hours) thereby inhibiting the use of the cryotherapy chamber 101 during this time. In a non-limiting embodiment, the use of hot gas bypass (HGP) coupled with an expansion tank for pressure regulation to defrost the evaporator coil rapidly via internal heat transfer may help to mitigate the problems associated with water vapor ingress into the cryotherapy chamber 101 including having ice on the evaporator coil. Low temperature refrigerants, such as, but not limited to, r508b or r744a, become highly pressured at even ambient temperatures. The use of HGB with these refrigerants is difficult without regulation of their pressure. Utilizing an expansion tank and valve to allow hot discharge gas to expand, thereby lowering pressure during the HGB cycle, is one method of defrosting an evaporator coil rapidly via internal heat transfer.

The cryotherapy system 100 may further include the use of artificial intelligent diagnostics and scheduling in order to reduce higher power consumption and costly downtime due to efficiency and labor-intensive diagnostics. The artificial intelligent diagnostics and scheduling system may include a machine learning algorithm that is capable of auto scheduling the ON/OFF times of the cryotherapy chamber 101 to ensure maximum efficiency and lowest power usage. Additionally, the machine learning algorithm can be used to predict issues with the cryotherapy chamber 101 before the issues occur thereby mitigating costly downtime.

FIG. 2 provides a diagram of an exemplary presence sensor 202 utilized in a presence sensor system 101 as described in part above with respect to FIG. 1. The presence sensor 202 is useful for indicating to a programmable logic controller/external controller 208 associated with the engine (e.g., engine 220) of the cryotherapy system 100 whether or not a user 203 is present within the cryotherapy chamber 101. Currently, administrative staff at a cryotherapy facility have to check physically to determine if the user 203 has stepped outside and left the cryotherapy chamber 101. There are a number of issues also with this current state of affairs in that administrative staff may not automatically know when the user 203 has left the cryotherapy chamber 101 in order to determine how to keep the cryotherapy chamber 101 at a right temperature and/or for energy conservation to power down or power off the evaporator fans 302a and 302b, as shown within FIG. 3, to an idle state within the cryotherapy chamber 101 when the user 203 is no longer located within the cryotherapy chamber 101. Beneficially, the presence sensor 202 is configured to automatically signal to the controller 208 when a user 203 is not present in the cryotherapy chamber 101 as shown in state 1 204 or when the user 203 is detected within the cryotherapy chamber 101 as shown in state 2 206 of FIG. 2. In a non-limiting embodiment, the presence sensor 202 is an infrared sensor capable of detecting infrared heat from the user 203. Infrared sensors, such as the presence sensor 202, are able to be operational at ultralow temperatures such as those reached in the cryotherapy chamber 101. The infrared presence sensor 202 is rather sensitive and able to detect any change in infrared state. Ultimately, if the infrared presence sensor 202 detects a vacancy, the systems within the cryotherapy chamber 101 can be triggered to automatically reset to a nominal default state. For example, if the presence sensor 202 detects a vacancy within the cryotherapy chamber 101 (i.e. no infrared signals indicating the presence of a user 203), all fans, including auxiliary windchill fans 304 and evaporator fans 302a, 302b, may be reset to a nominal state (0 percent state or lowest stage or off mode) in order to conserve energy and prevent unwanted operation of the fans 302a, 302b, 304, etc. at high states when there is an absence of a user 203 benefiting from the intense windchill and low temperatures inside of the cryotherapy chamber 101.

In alternative embodiments, the presence sensors 202 may be made up of floor sensors that provide digital signals to the controller 208. Alternatively, the presence sensors 202 may be made up of a laser such as an infrared laser that has a receiver. Accordingly, when someone passes through the laser, an indication is sent to the controller 208 of a person's presence. One or more coatings may need to be added to the presence sensor 202 in order to be operational at ultralow temperatures within the cryotherapy chamber 101. Further, the positioning of the presence sensor 202 within the cryotherapy chamber 101 is important so avoid having the presence sensor 202 detect infrared from other components within the cryotherapy chamber 101 such as the evaporator fans 302a and 302b and falsely indicate that a user 203 is present within the cryotherapy chamber 101 after detecting the infrared heat from such electronic components. In some cases, the presence sensor is positioned near the point of entry and exit or another location within the cryotherapy chamber 101 in order to be triggered by the movement of the user inside and outside of the cryotherapy chamber, which may include positioning the presence sensor on a door or wall of the cryotherapy chamber 101. Alternatively, the presence sensor 202 may be placed on a wall inside of the cryotherapy chamber 101 facing the user 203 but preferably not facing the evaporator fans 302a and 302b. Alternatively , the presence sensor 202 may be a floor sensor or placed anywhere within the cryotherapy chamber 101.

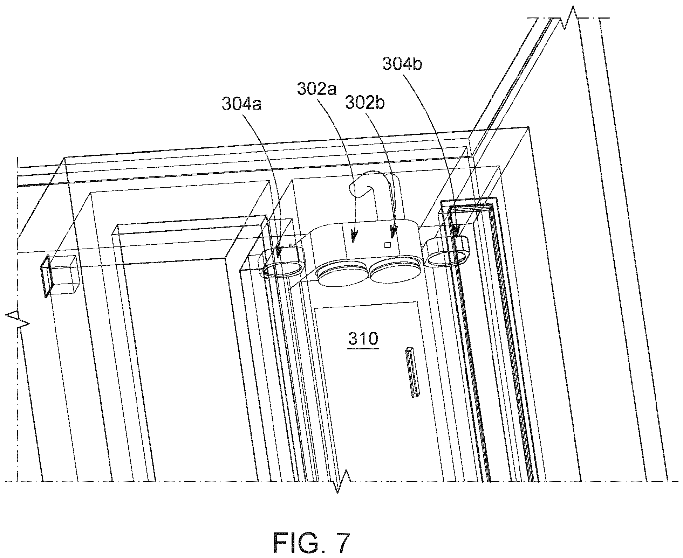

FIG. 3 provides a diagram of an exemplary prescriptive windchill system 104 for the user 203 to be enabled to change the effective temperature of the cryotherapy chamber 101 using internal controls, such as the user interface 306, located within the cryotherapy chamber 101 and accessible to the user 203 from within the cryotherapy chamber 101. As shown in the diagram in FIG. 3, when the user 203 is within the cryotherapy chamber 101, there may be one or more auxiliary windchill fans 304 that can be powered on and/or activated over a range of levels for the user 203 to increase the rate of airflow inside of the cryotherapy chamber 101 and thereby increase how intense the cold temperatures in the cryotherapy chamber 101 feel to the user 203. By increasing the windchill in the cryotherapy chamber 101 due to the activation of the auxiliary windchill fans 304 (e.g., auxiliary windchill fans 304a, 304b as shown in FIGS. 7-8), the heat transfer rate is effectively changing and it has aa substantial effect on how cold the cryotherapy chamber 101 feels to the user 101. It is very difficult to be able to dynamically shift the temperature inside of the cryotherapy chamber 101 quickly enough for those users 101 who would like the cryotherapy chamber 101 to be almost instantaneously colder when the user 101 is located within the cryotherapy chamber 101. It is noted that usually due to how sensitive a user's skin is to such ultralow temperatures (e.g., on the order of -90 degrees Fahrenheit to -160 degrees Fahrenheit), the cryotherapy session may only be a few minutes long (which does not provide a great amount of time to cause a change to the temperature of the cryotherapy chamber 101 for the user 203). Notably, having the auxiliary windchill fans 304 and the user interface 306 accessible to the user 203 from inside of the cryotherapy chamber 101 provides the user 203 with the freedom and the ability to dynamically change the effective air temperature during the user 203's session which has not been previously an option available to the user 203. As noted above, either the evaporator fans 302 operate at a constant, unchangeable rate or if there are controls to change the rate of the evaporator fans 302, these controls are positioned outside of the cryotherapy chamber 101, not inside as shown in FIG.3 for the prescriptive windchill system 104.

In a non-limiting embodiment, the user interface 306 is a frictional slide potentiometer that acts as a slider bar. The user 203 can slide the slider bar of the user interface 306 up or down to change the speed rate of the auxiliary windchill fan 304, and thereby increase windchill in the cryotherapy chamber 101 and causing the temperature to lower in the cryotherapy chamber 101. It may be preferably that a frictional slide potentiometer be used for the user interface 306 as the friction or heat helps to prevent the potentiometer from binding up in the ultralow temperatures of the cryotherapy chamber 101 and not being useful. In other non-limiting embodiments, there may be a number of selector buttons that each visually indicate a level or setting for setting the auxiliary fans 304 to using the selector buttons. Alternatively, a frictionless rotary encoder may also be used as the user interface 306. It is noted that there may be a number of alternative embodiments for the user interface 306 other than those discussed herein. Alternatively, the user interface controller 306 may be a smartwatch, smart tablet, goggles, knobs, buttons, or any other means of interaction to affect the speed or intensity of the auxiliary windchill fan 304.

The user interface 306 may be in electronic and in signal communication with the external controller 208 located within a main engine unit 220 that operates the cryotherapy chamber 101. In a non-limiting embodiment, the controller 208 may be a programmable logic controller (PLC) that receives data through its inputs and sends operating instructions through its outputs to control the operation of the cryotherapy chamber 101 and the cryotherapy system 100. Responsive to receive the data from the user interface 306, the controller 208 indicates to the windchill fans 304 to increase or decrease in speed. The PLC controller 208 affects the voltages and speeds that affect the auxiliary windchill fans 304 as well as the evaporator fans 302a, 302b.

Further in a non-limiting embodiment, the speed of the evaporator fans 302a, 302b can conversely be increased or decreased automatically to correspond with the selected speed of the auxiliary windchill fans 304, thus further increasing the windchill in the cryotherapy chamber 101 and how cold the temperature feels inside of the cryotherapy chamber 101 to the user 203. Advantageously, prescriptive windchill 104 is a shift in heat transfer rate inside of the cryotherapy chamber 101 by modifying the coefficient of convection, meaning that the prescriptive windchill system 104 increases the actual windspeed due to the presence of the auxiliary windchill fans 304. The windchill fans 304 are auxiliary fans that make a huge turbulence inside the overall system that increases the heat transfer rate within the cryotherapy chamber 101 in a short amount of time.

As noted above, the presence sensor system 102 shown in FIG. 2 may be combined with the prescriptive windchill system 104 shown in FIG. 3. For example, there may be a bypass function that allows the administrative entity and staff operating the cryotherapy chamber 101 to automatically turn down the prescriptive windchill fans 304 if the user 203 is detected (via the presence sensor 202 as shown in a non-limiting example in FIG. 2) to no longer be present in the cryotherapy chamber 101 and the prescriptive windchill fans 304 are left running on at low to full speed. A bypass function enables the PLC controller 208 to control remotely and externally the operation of the auxiliary windchill fans 304, but does not negate the ability for the user to also operate the auxiliary windchill fans 304 by using the user interface 306 when the user 203 is in a cryotherapy session in the cryotherapy chamber 101.

In a non-limiting embodiment, the internal controller/user interface controller 202 shown in FIG. 2 and the internal controller/user interface controller 306 shown in FIG. 3 may connect directly to the evaporator fans 302a, 302b, auxiliary fans 304 and cause a change in a voltage and/or frequency of the evaporator fans 302a, 302b, auxiliary fans 304, and other components in order to increase or decrease the speed of the evaporator fans 302a, 302b, and auxiliary fans 304.

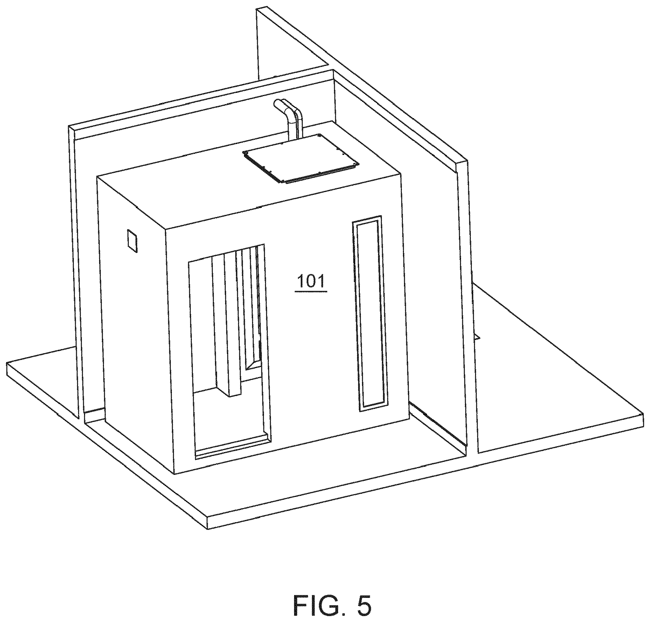

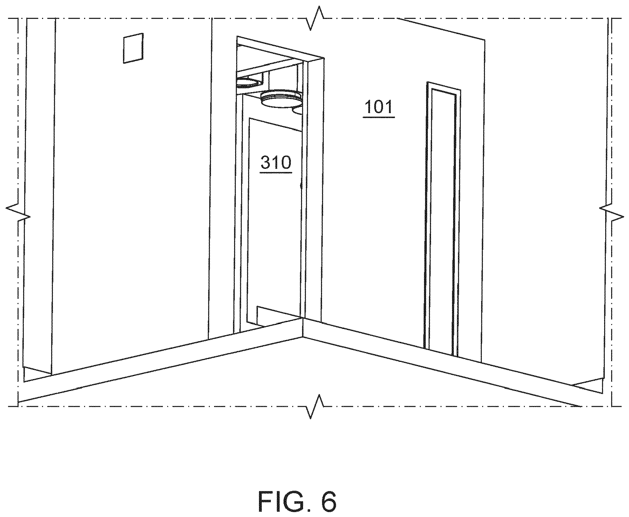

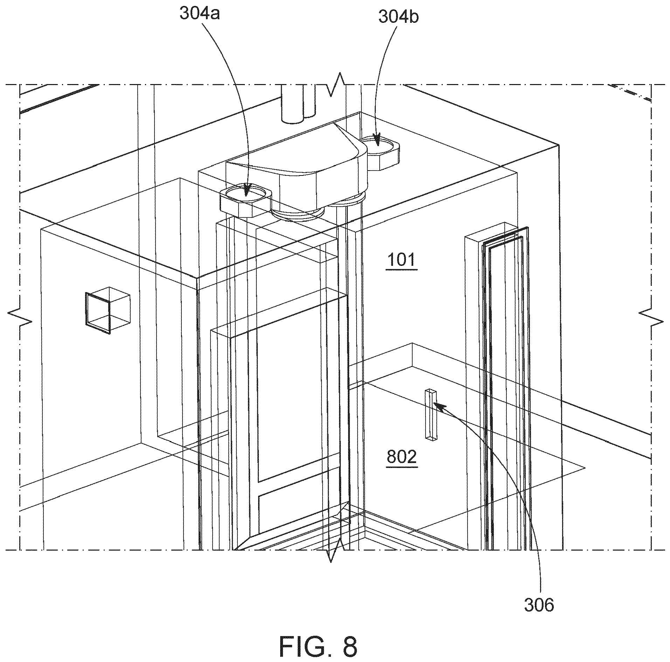

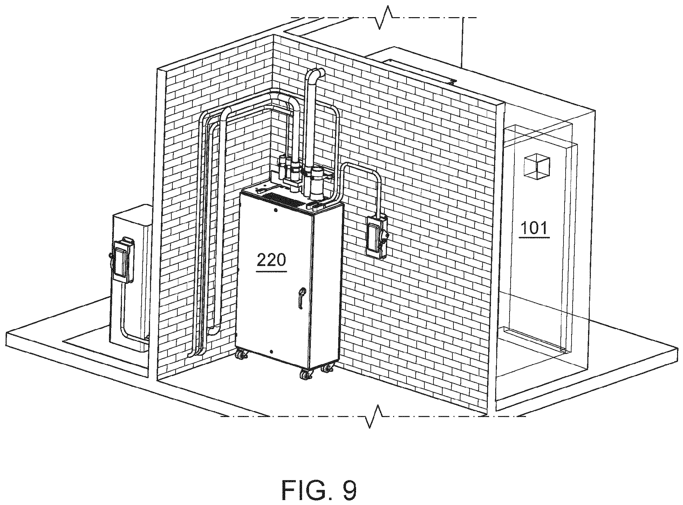

FIGS. 5-8 show pictorial illustrations of an exemplary cryotherapy chamber 101. Further, FIG. 6 shows a pictorial illustration of an exemplary cryoevaporator 310. FIG. 7 shows a pictorial illustration of exemplary evaporator fans 302a, 302b and one or more auxiliary windchill fans 304a, 304b positioned adjacent to or proximate to the evaporator fans 302a, 302b. It is noted that the auxiliary fans 304a, 304b that make up an important component of the prescriptive windchill system 104 may be placed elsewhere in the cryotherapy chamber 101 other than the placement shown herein. FIG. 8 displays an exemplary user interface 306 accessible on an interior wall 802 of the cryotherapy chamber 101 for the user 203 to selectively power on the auxiliary windchill fans 304a, 304b and select a level of speed for the auxiliary windchill fans 304a, 304b. FIG. 9 also provides an illustration of the engine unit 220. While not shown in FIG. 9, the engine unit 220 contains the PLC controller 208 that operates the prescriptive windchill system 104 and the presence sensor system 102.

Notably, the combination of the presence sensor system 102 and the prescriptive windchill system 104 may be useful to the administrative entity and staff operating the cryotherapy chamber 101.

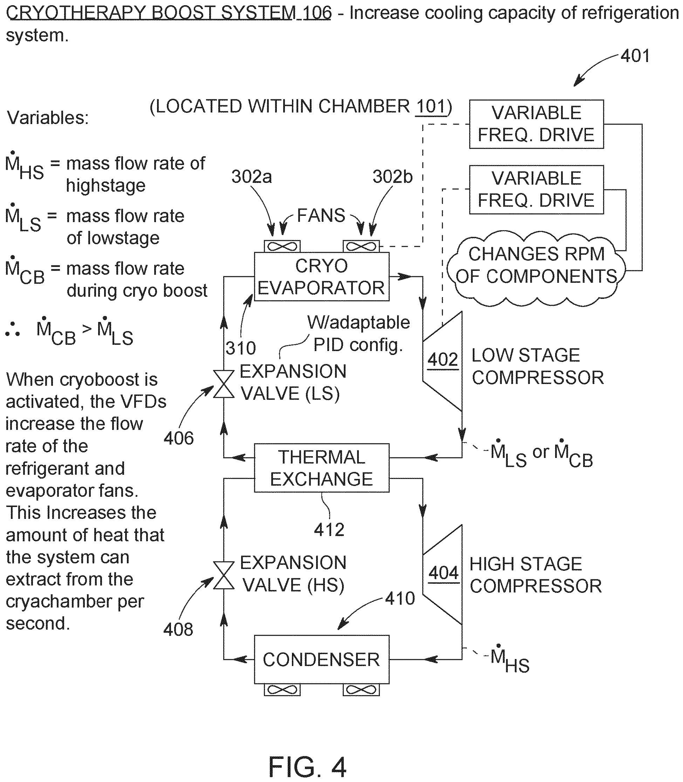

Turning to FIG. 4, FIG. 4 depicts a diagram describing some components that make up the cryotherapy boost system 106. The cryotherapy boost system 106 may incorporate the use of one or more variable frequency drives (VFDs) 401 in order to change the speed or revolutions per minute (RPMs) of the various components of the cryotherapy boost system 106. The VFDs 401 act as motor controllers coupled to the low stage compressor 402 as shown in FIG.4 that can dynamically and rapidly change the speed of the flowrate of refrigerant through the low stage compressor 402. In other words, VFD 401 changes the speed of compression of the refrigerant that flows through the low stage compressor 402.

The heat exchange system shown in FIG. 4, may further include one or more expansion valves 406, a thermal exchange unit 412, a high stage compressor 404, additional expansion valves 408, and a condenser 410. The heat exchange system shown in FIG. 4 is adapted to extract heat or remove heat from the cryotherapy chamber 101 in order to achieve ultralow temperatures inside of the cryotherapy chamber 101 that may be used for a cryotherapy treatment to the user 203.

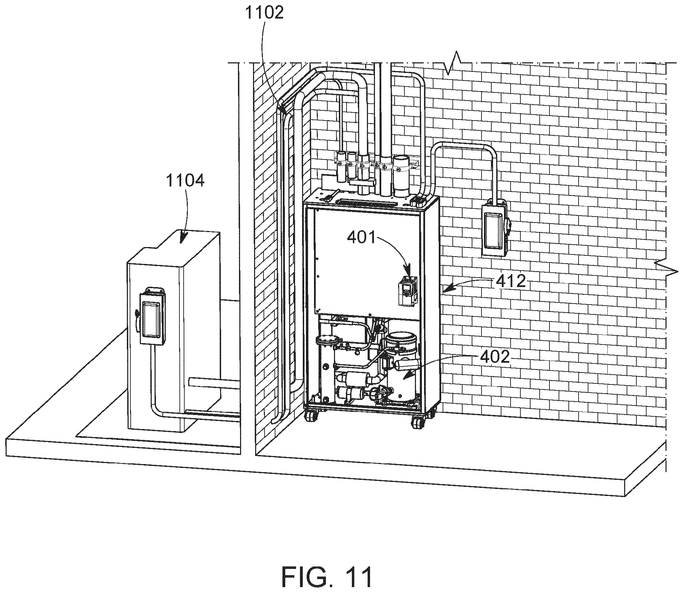

Because the temperatures required for the cryotherapy chamber 101 are so low, the cryotherapy system 100 may use at least two compressors, as shown in FIG. 4, with the integration of the low stage compressor 402 and the high stage compressor 404. Similar with other compressors, the refrigerant enters the low stage compressor 402 after traveling through the thermal exchange system 412 and then proceeds through one or more pipelines or conduits (e.g., conduits 1102 in FIG. 11) to the high stage compressor 404. The refrigerant enters both the low stage compressor 402 and the high stage compressor 404 as a low-pressure, low temperature gas and leaves the low stage compressor 404 and high stage compressor 404 as a high-pressure, high temperature gas.

The condenser 410 is a type of heat exchanger supplied with the high-temperature high-pressure, vaporized refrigerant coming off of the low stage compressor 402 and the high stage compressor 404 that is configured to receive the high-temperature, high-pressure, vaporized refrigerant coming off the low stage compressor 402 and the high stage compressor 404. The condenser 410 removes heat from the hot refrigerant vapor gas and condenses the hot refrigerant vapor gas into a saturated liquid state (i.e., condensation). After condensing, the refrigerant becomes a high-pressure, low temperature liquid, at which point the refrigerant in this form is routed to one or more expansion valves 406, 408. The expansion valves 406, 408 create a drop in pressure after the refrigerant leaves the condenser 410 which causes some of the refrigerant to quickly boil, creating a two-phase mixture called flashing, whereby the refrigerant enters the cryoevaporator 310. The cryoevaporator 310 absorbs heat when the refrigerant enters the cryoevaporator 310 as a low temperature liquid at low pressure, and the cryoevaporator fans 302a, 302b force air across fins of the cryoevaporator 310, thereby cooling the air in the cryotherapy chamber 101. After doing so, the refrigerant is sent back to the compressors 402, 404 for the refrigeration loop to begin again.

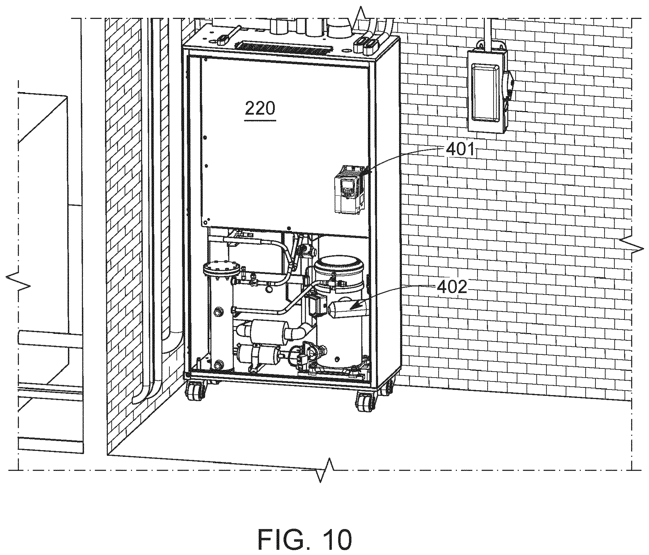

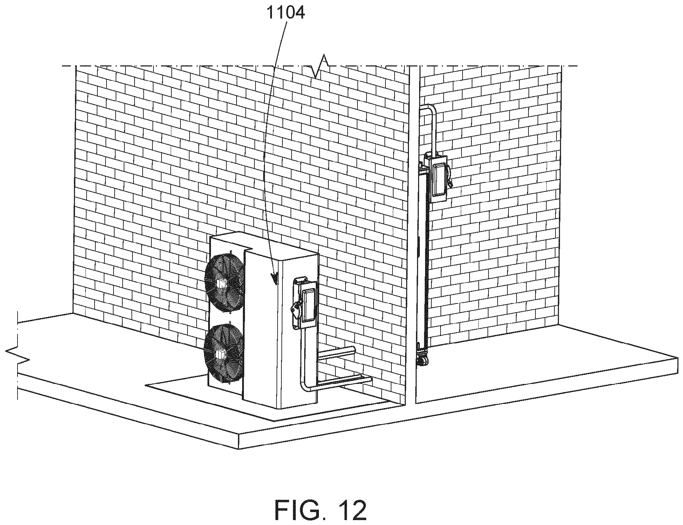

FIGS. 9-12 illustrate some of the exemplary components of the cryotherapy boost system 106, as well as components relevant to the prescriptive windchill system 104 and the presence sensor 102 system. As shown in FIG. 10, the main engine 220 may include a low stage compressor 402 as well as the variable frequency drive(VFD) 401 used to manipulate the speed of flowrate of refrigerant flowing through the low stage compressor 402. As noted above, the PLC controller 208 may also be located in the main engine 220 unit as shown in FIG. 10. Through one or more conduits shown in FIG. 10 and in FIG. 11, the refrigerant flows through the thermal exchange 412 shown in FIG. 4 and in FIG. 11 and through one or more conduits 1102 to an externally positioned unit 1104. The external unit 1104 may house the high stage compressor 404 as well as the condenser 410. In a non-limiting embodiment, the heat in the cryotherapy chamber 101 travels from the cryotherapy chamber 101 through the main engine 220 and then through the condenser 410 located outside the building or facility that the cryotherapy chamber 101 is located within.

Advantageously, when the cryotherapy boost system 106 is activated, the VFDs 401 increase the flowrate of the refrigerant flowing through the refrigerant loop (e.g., as shown in FIG. 4) and also as cooled by the evaporator fans 302a, 302b. The cryotherapy boost system 106 increases the amount of heat that the cryotherapy boost system 106 can extract from the cryotherapy chamber 101 per second, which is very important as the therapy session for most users 203 lasts only a few minutes.

In a non-limiting embodiment, the prescriptive windchill system 104 works in conjunction with the cryotherapy boost system 106 to further enhance the ability of the cryotherapy chamber 101 to cool down quickly to ultralow temperatures. Accordingly, the cryotherapy boost system 106 may be configured such that if the windchill fans 304 are activated via the user 203 selection of controls or a controller on the user interface 306 (e.g., as shown in FIG. 3), the cryotherapy boost system 106 may be programmed to trigger on automatically as well and to further cause the cryotherapy chamber 101 to cool even quicker. Notably, the cryotherapy boost system 106 comprises a variable frequency drive (VFD) 401 to increase the flowrate of refrigerant flowing through the heat transfer system of the cryotherapy chamber 101 and the flowrate of evaporator fans 302a, 302b in the cryoevaporator to increase an amount of heat extracted from the cryochamber 101 per second.

Advantageously, the prescriptive windchill system 104 allows a user to dynamically affect windchill and effective temperature of the cryotherapy chamber 101 from within the cryotherapy chamber 101. Further, the cryotherapy boost system 106 on its own is able to increase the cooling capacity of the refrigeration system of the cryotherapy chamber 101 with the incorporation of one or more VFDs 401. Additionally, the presence sensor system 102 indicates when the user 203 is located inside of the cryotherapy chamber 101 or not so that automatic adjustments may be made if necessary to the cryotherapy chamber 101 from outside of the cryotherapy chamber 101. The combination of all of these systems 102, 104, and 106 functions to provide a better cryotherapy treatment session to the user 203 and addresses a number of deficiencies of existing cryotherapy chambers 101 as noted above. Advantageously, the user can change the intensity of their cryotherapy session, whether that involves changing the intensity of the windchill through user control of the evaporator fans 302a,302b and the auxiliary fans 304 as shown in FIG. 3, and/or by changing the flowrate of the refrigerant as shown in FIG. 4, the user has a greater and much desired ability through these systems to affect the performance of their individualized cryotherapy. The user greatly desires the ability to enter a fully enclosed cryochamber 101 and fully affect the windchill and temperature via the controls offered herein by these systems, including but not limited to, the prescriptive windchill system 104, the cryotherapy boost system 106, and/or the use of the presence sensor 102. Currently the manufacturers have a predefined temperature driven by the manufacturers that are not alterable internally by the users. The present description includes one or more non-limiting embodiments to combat this existing problem.

In the Summary above and in this Detailed Description, and the claims below, and in the accompanying drawings, reference is made to particular features (including method steps) of the invention. It is to be understood that the disclosure of the invention in this specification includes all possible combinations of such particular features. For example, where a particular feature is disclosed in the context of a particular aspect or embodiment of the invention, or a particular claim, that feature can also be used, to the extent possible, in combination with and/or in the context of other particular aspects and embodiments of the invention, and in the invention generally.

The term "comprises" and grammatical equivalents thereof are used herein to mean that other components, ingredients, steps, among others, are optionally present. For example, an article "comprising" (or "which comprises") components A, B, and C can consist of (i.e., contain only) components A, B, and C, or can contain not only components A, B, and C but also contain one or more other components.

Where reference is made herein to a method comprising two or more defined steps, the defined steps can be carried out in any order or simultaneously (except where the context excludes that possibility), and the method can include one or more other steps which are carried out before any of the defined steps, between two of the defined steps, or after all the defined steps (except where the context excludes that possibility).

The term "at least" followed by a number is used herein to denote the start of a range beginning with that number (which may be a range having an upper limit or no upper limit, depending on the variable being defined). For example, "at least 1" means 1 or more than 1. The term "at most" followed by a number is used herein to denote the end of a range ending with that number (which may be a range having 1 or 0 as its lower limit, or a range having no lower limit, depending upon the variable being defined). For example, "at most 4" means 4 or less than 4, and "at most 40%" means 40% or less than 40%. When, in this specification, a range is given as "(a first number) to (a second number)" or "(a first number) - (a second number)," this means a range whose lower limit is the first number and whose upper limit is the second number. For example, 25 to 100 mm means a range whose lower limit is 25 mm and upper limit is 100 mm.

Certain terminology and derivations thereof may be used in the following description for convenience in reference only and will not be limiting. For example, words such as "upward," "downward," "left," and "right" would refer to directions in the drawings to which reference is made unless otherwise stated. Similarly, words such as "inward" and "outward" would refer to directions toward and away from, respectively, the geometric center of a device or area and designated parts thereof. References in the singular tense include the plural, and vice versa.

The corresponding structures, materials, acts, and equivalents of all means or step plus function elements in the claims below are intended to include any structure, material, or act for performing the function in combination with other claimed elements as specifically claimed. The description of the present invention has been presented for purposes of illustration and description but is not intended to be exhaustive or limited to the invention in the form disclosed. Many modifications and variations will be apparent to those of ordinary skill in the art without departing from the scope and spirit of the invention.

The embodiments were chosen and described in order to best explain the principles of the invention and the practical application, and to enable others of ordinary skill in the art to understand the invention for various embodiments with various modifications as are suited to the particular use contemplated. The present invention according to one or more embodiments described in the present description may be practiced with modification and alteration within the spirit and scope of the appended claims. Thus, the description is to be regarded as illustrative instead of restrictive of the present invention.

## Claims

1. A system, comprising:
a cryotherapy chamber comprising:
a cryoevaporator comprising at least one evaporator fan ;
a prescriptive windchill system comprising:
at least one auxiliary windchill fan coupled to a user interface controller, wherein the user interface controller is accessible to a user from within an interior of the cryotherapy chamber and wherein the at least one auxiliary windchill fan is located within the interior of the cryotherapy chamber, wherein the user interface controller is selectable by the user to automatically activate and adjust a level of speed of the at least one auxiliary windchill fan; and
an external engine comprising a programmable logic controller (PLC) that controls and is in signal communication and in electronic communication with the user interface controller;
a presence sensor system comprising:
at least one presence sensor positioned within the cryotherapy chamber, wherein if the at least one presence sensor detects a vacancy inside of the cryotherapy chamber, a signal is sent to the PLC to return the at least one auxiliary windchill fan to a default state that comprises reducing a speed of the at least one auxiliary windchill fan or turns of the at least one auxiliary windchill fan.

2. The system of claim 1, further comprising a cryotherapy boost system, the cryotherapy boost system further comprising a variable frequency drive (VFD) that causes the flowrate of refrigerant to increase through a heat transfer system that is part of an operation of the cryotherapy chamber, thereby increasing an amount of heat that is extracted from the cryochamber per second.

3. The system of claim 2, wherein the heat transfer system comprises a heat exchanger, one or more exchange valves, a low stage compressor, a high stage compressor, and at least one condenser.

4. The system of claim 3, wherein a mass flow rate of the refrigerant during operation of the system is greater than a mass flow rate of the refrigerant of the low stage compressor.

5. The system of claim 1, wherein the presence sensor is an infrared sensor that detects infrared heat of the user.

6. The system of claim 1, wherein the user interface is a frictional slide potentiometer.

7. The system of claim 2, wherein activation of the at least one auxiliary windchill fan at a minimum level further activates the cryotherapy boost system to achieve a lower temperature within the cryotherapy chamber.

8. The system of claim 1, wherein the at least one presence sensor is positioned inside of the cryotherapy chamber near a door near the entrance and/or exit of the cryotherapy chamber.

9. The system of claim 1, wherein the at least one presence sensor is a floor sensor positioned on a floor of the cryotherapy chamber.

10. The system of claim 1, wherein the user interface controller comprises knobs, buttons, and/or sliders, and/or smart watches, tablets, and/or digital interfaces.

11. A system, comprising:
a cryotherapy chamber comprising:
a cryoevaporator comprising at least one evaporator fan; and
a prescriptive windchill system comprising:
at least one auxiliary windchill fan coupled to a user interface controller, wherein the user interface controller is accessible to a user from within an interior of the cryotherapy chamber and wherein the at least one auxiliary windchill fan is located within the interior of the cryotherapy chamber, wherein the user interface controller is selectable by the user to automatically activate and adjust a level of speed of the at least one auxiliary windchill fan and the at least one evaporator fan.

12. The system of claim 11, further comprising, a presence sensor system comprising at least one presence sensor positioned within the cryotherapy chamber, wherein if the at least one presence sensor detects a vacancy inside of the cryotherapy chamber, a signal is sent to return the at least one auxiliary windchill fan and the at least one evaporator fan to a default state that comprises reducing a speed of the at least one auxiliary windchill fan or turns of the at least one auxiliary windchill fan.

13. The system of claim 12, further comprising, a variable frequency drive (VFD) that causes the flowrate of refrigerant to increase through a heat transfer system that is part of an operation of the cryotherapy chamber, thereby increasing an amount of heat that is extracted from the cryochamber per second.

14. The system of claim 13, wherein the heat transfer system comprises a heat exchanger, one or more exchange valves, a low stage compressor, a high stage compressor, and at least one condenser.

15. The system of claim 13, wherein a mass flow rate of the refrigerant during operation of the system is greater than a mass flow rate of the refrigerant of the low stage compressor.
